(19) Europäisches Patentamt
European Patent Office
Office européen des brevets

(11) Veröffentlichungsnummer: **0 358 092 B1**

(12) **EUROPÄISCHE PATENTSCHRIFT**

(45) Veröffentlichungstag der Patentschrift: **11.12.91 Patentblatt 91/50**

(51) Int. Cl.[5]: **C07D 209/96**, C07C 255/46, C07C 229/28

(21) Anmeldenummer: **89115938.6**

(22) Anmeldetag: **29.08.89**

# (54) 2-Aza-4-(alkoxycarbonyl)spiro-[4,5]decan-3-on.

(30) Priorität: **01.09.88 CH 3272/88**
**12.04.89 CH 1382/89**
**12.04.89 CH 1383/89**

(43) Veröffentlichungstag der Anmeldung: **14.03.90 Patentblatt 90/11**

(45) Bekanntmachung des Hinweises auf die Patenterteilung: **11.12.91 Patentblatt 91/50**

(84) Benannte Vertragsstaaten: **AT BE CH DE ES FR GB IT LI NL SE**

(56) Entgegenhaltungen:
**DE-A- 2 317 180**
**DE-A- 2 460 891**
**DE-A- 2 541 855**
**DE-A- 2 611 690**
**GB-A- 1 415 338**
**CHEMICAL ABSTRACTS Band 57, Nr. 8, 15. Oktober 1962, Seite 1962, Spalte 9778f-9778g, Columbus, Ohio, USA; J. COLOGNE et al.: "Preparation of 2-pyrrolidones and Gamma-amino acids"**
**CHEMICAL ABSTRACTS Band 98, Nr. 1, 3. Januar 1983, Seite 392, Spalte 2, Zusammenfassung Nr. 4394c, Columbus, Ohio, USA: & JP - A - 57 102 854**

(56) Entgegenhaltungen:
**CHEMICAL ABSTRACTS Band 98, Nr. 3, 17. Januar 1983, Seite 475, Spalte 1, Zusammenfassung Nr. 16297m, Columbus, Ohio, USA; & JP - A - 57 102 851**
**CHEMICAL ABSTRACTS Band 98, Nr. 7, 14. Februar 1983, Seite 607, Spalte 2, Zusammenfassung Nr. 53180p, Columbus, Ohio, USA; & JP - A - 57 102 850**

(73) Patentinhaber: **LONZA A.G.**
**Münchensteinerstrasse 38**
**CH-4002 Basel Postfach (CH)**

(72) Erfinder: **Mettler, Hans Peter, Dr.**
**Kirchweg 16**
**Brig-Glis (Kanton Wallis) (CH)**
Erfinder: **Griffiths, Gareth, Dr.**
**Bäretstrasse 6**
**Visp (Kanton Wallis) (CH)**
Erfinder: **Mills, Lester, Dr.**
**Furkastrasse 65**
**Naters (Kanton Wallis) (CH)**
Erfinder: **Previdoli, Felix, Dr.**
**Rhonesandstrasse 24**
**Brig (Kanton Wallis) (CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40 (DE)**

**EP 0 358 092 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft 2-Aza-4-(alkoxycarbonyl)spiro[4,5]-decan-3-one und ein Verfahren zur Herstellung derselben. 2-Aza-4-(alkoxycarbonyl)spiro[4,5]decan-3-one sind wertvolle Ausgansprodukte zur Herstellung des Anticonvulsivums Gabapentin (1-(Aminomethyl)cyclohexanessigsäure).

Gabapentin sowie seine Herstellung werden in Drugs of the Future, Vol. 9, Nr. 6, 1984 S. 418-419 sowie in den US Patenten 4024175 und 4152326 beschrieben. Die hier beschriebene Herstellung verläuft über 7 bis 8 Stufen und ist relativ aufwendig.

Ziel der vorliegenden Erfindung ist es, neue Produkte zur Verfügung zu stellen, die geeignet sind, auf einfache Weise die 1-(Aminomethyl)cyclohexanessigsäure zu gewinnen.

Erfindungsgemäss wird dies durch die in den Patentansprüchen 1 und 2 beanspruchten Produkte erreicht.

Die neuen Produkte der Erfindung werden nach einem Verfahren gemäss Patentansprüchen 5 bis 11 hergestellt.

Das für das Verfahren der Erfindung verwendete Ausgangsprodukt (2) kann auf einfache und bekannte Weise durch eine Knoevenagel'sche Kondensationsreaktion aus Cyclohexanon und Alkylmalonat hergestellt werden.

Der so hergestellte Cyclohexylidenmalonsäurealkylester (2) wird in einer ersten Stufe entweder mit Blausäure, in Gegenwart katalytischer Mengen eines Alkalicyanids, oder mit einer stöchiometrischen Menge Alkalicyanid, in einem Alkohol, und anschliessender Zugabe einer Säure, in den (1-Cyanocyclohexyl)malonsäuredialkylester (3) überführt.

Die Menge HCN beträgt 1 bis 20 Aequivalente, vorzugsweise 3 bis 5 Aequivalente bezogen auf Ausgangsprodukt (2).

Die Menge an Alkalicyanid beträgt 0,1 bis 50 mol%, vorzugsweise 10 bis 20 mol% bezogen auf Ausgangsprodukt (2).

Als Alkalicyanid kann Na- oder K-cyanid verwendet werden, vorzugsweise wird Kaliumcyanid verwendet.

Die Umsetzung kann ohne Lösungsmittel oder mit Lösungsmittel durchgeführt werden. Als Lösungsmittel können niedere Alkohole, wie Methanol, Ethanol, Propanol, Butanol oder Alkohol/Wassergemische, Ester mit niederen Alkoholen in der Estergruppe, wie Ethylacetat, Methylacetat, Propylacetat, Ketone, wie Aceton, Methylethylketon, angewendet werden.

Vorzugsweise wird die Umsetzung ohne Lösungsmittel durchgeführt.

Die Umsetzung in der ersten Stufe wird bei Temperaturen von 20 bis 150°C, vorzugsweise bei 90 bis 120°C durchgeführt, wobei vorzugsweise ein geschlossenes Gefäss (Autoklav) Verwendung findet und sich jeweils der Druck, gegeben durch die Reaktionstemperatur, einstellt.

Die Menge Alkalicyanid (zweite Möglichkeit) beträgt 1 bis 5 Aequivalente, vorzugsweise 1 bis 1,5 Aequivalente, bezogen auf das Ausgangsprodukt (2), wenn der pH-Wert des Gemisches durch kontinuierliche Zugabe von Säure/Alkohol bei einem Wert von 10,0 bis 13,0 gehalten wird.

Als Alkalicyanid kann Na- oder K-cyanid verwendet werden, vorzugsweise wird Kaliumcyanid verwendet.

Als Säuren können Mineralsäuren, wie beispielsweise Salzsäure oder Schwefelsäure, oder organische Säuren, wie beispielsweise Ameisensäure oder Essigsäure angewendet werden, vorzugsweise wird Salzsäure angewendet.

Als Lösungsmittel können niedere Alkohole, wie Methanol, Ethanol, Propanol, Butanol, oder Alkohol/Wassergemische angewendet werden.

Vorzugsweise wird die Umsetzung mit dem der Alkylestergruppe entsprechenden Alkohol durchgeführt.

Die Reaktionstemperatur liegt bei 0°C bis Rückflusstemperatur, vorzugsweise bei Rückflusstemperatur.

Der nach der ersten Stufe gebildete (1-Cyanocyclohexyl)malonsäuredialkylester (3) wird in einer zweiten Stufe durch katalytische Hydrierung in das 2-Aza-4-(alkoxycarbonyl)spiro[4,5]-decan-3-on überführt.

Als Katalysator können Raney-Nickel, Raney-Kobalt oder Edelmetallkatalysatoren, wie Platin, Palladium, Rhodium oder Ruthenium auf Träger, wie z.B. Kohle, eingesetzt werden.

Die Katalysatormenge, bei bevorzugter Verwendung von Raney-Nickel, liegt zweckmässig bei 1 bis 50 Gew.%, bezogen auf Ausgangsprodukt (3).

Als Lösungsmittel kommen niedrige Alkohole, wie Ethanol oder andere polare Lösungsmittel, wie Ester oder Ether, zur Anwendung.

Die Reaktionstemperatur liegt bei 20 bis 150°C, vorzugsweise bei 80 bis 100°C.

Der zur Anwendung gelangende $H_2$-Druck liegt bei 1 bis 100 bar, vorzugsweise bei 5 bis 10 bar.

Das für das Verfahren der Erfindung verwendete Ausgangsprodukt (4) kann ebenfalls auf einfache und bekannte Weise durch eine Knoevenagel'sche Kondensationsreaktion aus Cyclohexanon und Cyanalkylat hergestellt werden.

Das so hergestellte Cyclohexylidencyanalkylat (4) wird in einer ersten Stufe entweder mit Blausäure, in

Gegenwart katalytischer Mengen eines Alkalicyanides oder mit einer stöchiometrischen Menge Alkalicyanid in Alkohol, in den (1-Cyanocyclohexyl)cyanessigsäurealkylester (5) überführt.

Die Menge HCN beträgt 1 bis 20 Aequivalente, vorzugsweise 3 bis 5 Aequivalente bezogen auf Ausgangsprodukt (4).

Die Menge an Alkalicyanid beträgt 0,1 bis 50 mol%, vorzugsweise 10 bis 20 mol% bezogen auf Ausgangsprodukt (4).

Als Alkalicyanid kann Na- oder K-cyanid verwendet werden, vorzugsweise wird Kaliumcyanid verwendet.

Die Umsetzung kann ohne Lösungsmittel oder mit Lösungsmittel durchgeführt werden. Als Lösungsmittel können niedere Alkohole, wie Methanol, Ethanol, Propanol, Butanol oder Alkohol/Wassergemische, Ester mit niederen Alkoholen in der Estergruppe, wie Ethylacetat, Methylacetat, Propylacetat, Ketone, wie Aceton, Methylethylketon, angewendet werden.

Vorzugsweise wird die Umsetzung ohne Lösungsmittel durchgeführt.

Die Umsetzung in der ersten Stufe wird bei Temperaturen von 20 bis 150°C, vorzugsweise bei 90 bis 120°C durchgeführt, wobei vorzugsweise ein geschlossenes Gefäss (Autoklav) Verwendung findet und sich jeweils der Druck, gegeben durch die Reaktions temperatur, einstellt.

Die Menge Alkalicyanid (zweite Möglichkeit) beträgt von 1 bis 5 Aequivalenten, vorzugsweise von 1 bis 1,5 Aequivalenten, bezogen auf Ausgangsprodukt (4).

Als Lösungsmittel können niedere Alkohole wie Methanol, Ethanol, Propanol und Butanol angewendet werden.

Vorzugsweise wird die Umsetzung mit dem der Alkylestergruppe entsprechenden Alkohol durchgeführt.

Der nach der ersten Stufe gebildete (1-Cyanocyclohexyl)cyanessigsäurealkylester (5) wird in einer zweiten Stufe, vorzugsweise in einem geschlossenen Gefäss, mit 1 bis 100 Aequivalenten einer Säure, vorzugsweise mit 10 bis 20 Aequivalenten bezogen auf das Ausgangsprodukt (5), bei einer Temperatur von –20 bis 50°C und einem Druck von 1 bis 10 bar, vorzugsweise bei 0 bis 20°C und einem Druck von 2 bis 3 bar, in den (1-Cyanocyclohexyl)malonsäuredialkylester (3) überführt.

Als Säuren können Mineralsäuren, wie beispielsweise Salzsäure oder Schwefelsäure, sowie organische Säuren, wie beispielsweise Ameisensäure, oder Essigsäure angewendet werden, vorzugsweise wird Salzsäure angewendet.

Als Lösungsmittel kommen die der Alkylestergruppe entsprechenden niedrigen Alkohole, alleine oder in Verbindung mit einem Ether, Kohlenwasserstoff, beispielsweise Toluol oder Hexan, oder einem halogenierten Kohlenwasserstoff, beispielsweise Methylenchlorid, zur Anwendung.

Vorzugsweise wird die Umsetzung zum Ethylester mit Ethanol durchgeführt.

Der nach der zweiten Stufe gebildete (1-Cyanocyclohexyl)malonsäuredialkylester (3) wird in einer dritten Stufe durch katalytische Hydrierung in das 2-Aza-4-(alkoxycarbonyl)spiro[4,5]-decan-3-on überführt.

Als Katalysator können Raney-Nickel, Raney-Kobalt oder Edelmetallkatalysatoren, wie Platin, Palladium, Rhodium oder Ruthenium auf Träger, wie z.B. Kohle, eingesetzt werden.

Die Katalysatormenge, bei bevorzugter Verwendung von Raney-Nickel, liegt zweckmässig bei 1 bis 50 Gew.%, bezogen auf Ausgangsprodukt (3).

Als Lösungsmittel kommen niedrige Alkohole, wie Ethanol oder andere polare Lösungsmittel, wie Ester oder Ether, zur Anwendung.

Die Reaktionstemperatur liegt bei 20 bis 150°C, vorzugsweise bei 80 bis 100°C.

Der zur Anwendung gelangende $H_2$-Druck liegt bei 1 bis 100 bar, vorzugsweise bei 5 bis 10 bar.

Die neuen 2-Aza-4-(alkoxycarbonyl)spiro[4,5]decan-3-one lassen sich auf bekannte Weise durch Hydrolyse mit HCl oder Schwefelsäure bei erhöhten Temperaturen von etwa 50 bis 200°C in das entsprechende Gabapentinsalz überführen. Wird HCl verwendet, z.B. als 20%ige Lösung in Wasser, wird das entsprechende Gabapentinhydrochlorid anfallen.

Beispiel 1

Cyclohexylidenmalonsäuredimethylester (nicht erfindungsgemäss)

Während 65 Minuten wurde eine Lösung von Titantetrachlorid (95,1 g, 0,5 mol) in Tetrachlorkohlenstoff (125 ml) zu Tetrahydrofuran (1000 ml) bei ca. 0°C unter Stickstoff dosiert. Anschliessend wurde ein Gemisch von Cyclohexanon (24,6 g, 0,25 mol) und Malonsäuredimethylester (33,0 g, 0,25 mol) während 15 Minuten bei ca. 0°C hinzugegeben. Zu der gelben Suspension wurde Pyridin (79,0 g, 1,0 mol) in THF (175 ml) während 60 Minuten gegeben und das Gemisch wurde 18 Stunden bei Raumtemperatur weitergerührt. Wasser (250 ml) wurde hinzugegeben und die zwei Phasen wurden getrennt. Die wässrige Phase wurde zweimal mit je 90 ml Ethylacetat extrahiert und die vereinigten organischen Phasen wurden mit ges. Natriumchlorid- und ges.

Natriumcarbonatlösung (jeweils 100 ml) gewaschen, mit Magnesiumsulfat getrocknet, filtriert und eingedampft. Destillation (81 bis 83°C/1 mbar) ergab 22,5 g Produkt, entsprechend 43% Ausbeute (bez.einges.Malonat).

Beispiel 2

(1-Cyanocyclohexyl)malonsäuredimethylester

Ein Gemisch von Cyclohexylidenmalonsäuredimethylester (21,5 g, 94 mmol), Blausäure (19 ml, 485 mmol) und Kaliumcyanid (0,92 g, 14 mmol) wurde im Autoklav während 6 Stunden auf 120°C aufgeheizt. Nach Abkühlen auf Raumtemperatur wurde die überschüssige Blausäure mit Stickstoff ausgetrieben. Das Rohprodukt wurde in Ethylacetat gelöst und filtriert. Das eingedampfte Filtrat (24,2 g) wurde aus Ethanol umkristallisiert und ergab 15,5 g Produkt, entsprechend einer Ausbeute von 69% (bez.einges.Malonat).
Smp. 74 bis 75°C.
$^1$H-NMR : ($CDCl_3$, 300 MHz) δ

| | |
|---|---|
| 1,0-2,3 | (m, 10 H) |
| 3,47 | (s, 1 H) |
| 3,81 | (s, 6 H) |

Elementaranalyse für $C_{12}H_{17}NO_4$ (239, 3) :

| | |
|---|---|
| ber. | C 60,2% H 7,2% N 5,9% |
| gef. | C 60,6% H 7,3% N 6,5% |

Beispiel 3

2-Aza-4-(methoxycarbonyl)spiro[4,5]decan-3-on (1)

Eine Lösung von (1-Cyanocyclohexyl)malonsäuredimethylester (7,50 g, 29 mmol) in Ethanol (150 ml) wurde bei 10 bar Wasserstoffdruck und 90°C über 3,00 g Raney-Nickel während 4,5 Stunden hydriert. Das Reaktionsgemisch wurde filtriert, das Filtrat eingeengt und getrocknet. Der Rückstand wurde mit 5 g heissem Toluol versetzt, mit einigen Produktkristallen angeimpft und 4 Stunden bei 4°C stehengelassen. Die gebildeten Kristalle wurden abfiltriert, mit Toluol gewaschen und getrocknet. Man erhielt 4,13 g Produkt entsprechend einer Ausbeute von 66% (bez.einges.Malonat).
Smp. 73 bis 75°C.
$^1$H-NMR : ($CDCl_3$, 300 MHz) δ

| | |
|---|---|
| 1,28-1,71 | (m, 10 H) |
| 3,10 | (s, 1 H) |
| 3,20 | (d, J=10Hz, 1 H) |
| 3,36 | (d, J=10 Hz, 1H) |
| 3,78 | (s, 3H) |
| 6,89 | (s, 1 H) |

Elementaranalyse für $C_{11}H_{17}NO_3$ (211,3) :

| | |
|---|---|
| ber. | C 62,5% H 8,1% N 6,6% |
| gef. | C 62,9% H 8,3% N 7,2% |

Beispiele 4 und 5

Gemäss Beispielen 1 bis 3 wird der entsprechende (1-Cyanocyclohexy)malonsäurediethylester und das 2(Aza-4-ethoxycarbonylspiro[4,5]decan-3-on hergestellt. Die Ausbeuten entsprechen denen in Beispielen 1 bis 3 genannten.

(1-Cyanocyclohexyl)malonsäure-diethylester (Beispiel 4)

Smp. 90 bis 92°C

$^1$H-NMR : ($CDCL_3$, 300 MHz) δ

| | |
|---|---|
| 1.30 | (t, J=7,2 Hz, 6 H) |
| 1,15-2,23 | (m, 10 H) |
| 3,40 | (s, 1 H) |
| 4,20-4,35 | (m, 4 H) |

Elementaranalyse für $C_{14}H_{21}NO_4$(267, 3) :

ber. C 62,9 H 7,9 N 5,2
gef. C 62,9 H 7,9 N 5,5

2-Aza-4-(ethoxycarbonyl)spiro[4,5]decan-3-on (Beispiel 5)
Smp. 72 bis 74°C

$^1$H-NMR : ($CDCl_3$, 300 MHz) δ

| | |
|---|---|
| 1,29 | (t, J=7,2 Hz, 3 H) |
| 1,25-1,68 | (m, 10 H) |
| 3,06 | (s, 1 H) |
| 3,18 | (dxd, $J_1$ = 9,5 Hz, $J_2$ = 1,1 Hz, 1 H) |
| 3,34 | (d, J = 9,5 Hz, 1 H) |
| 4,21 | (q, J = 7,1 Hz, 2 H) |
| 6,92 | (s, 1 H) |

Elementaranalyse für $C_{12}H_{19}NO_3$ (225,3) :

ber. C 64,0% H 8,5% N 6,2%
gef. C 63,6% H 8,6% N 6,4%

Beispiel 6

(1-Cyanocyclohexyl)malonsäurediethylester

Ein Gemisch von Kaliumcyanid (18,1 g, 0,27 mol) und Cyclohexylidenmalonsäurediethylester (44,0 g, 0,18 mol) in Ethanol (180 ml) wurde zum Rückfluss erhitzt, wobei der pH-Wert des Gemisches durch kontinuierliche Zugabe von HCl/Ethanol bei einem Wert von 10,5 bis 11,5 gehalten wurde.

Nach einer Reaktionszeit von 16 h wurde auf 30°C abgekühlt und durch Zugabe von HCl/EtOH (24%ig, ca. 14 g, 0,1 mol) ein pH-Wert von ca. 5 eingestellt.

Das ausgefallene Kaliumchlorid wurde abfiltriert und mit Ethanol (200 ml) gewaschen.

Das eingedampfte Filtrat wurde aus Ethanol umkristallisiert und ergab total 42,1 g Produkt, entsprechend einer Ausbeute von 88%, bezogen auf eingesetzten Cyclohexylidenmalonsäureethylester.

Beispiel 7

Cyclohexylidencyanacetat (nicht erfindungsgemäss)

Eine Lösung von Cyclohexanon (58,9 g, 0,6 mol), Cyanessigsäureethylester (3,8 g, 0,5 mol), Ammoniumacetat (3,8 g, 0,05 mol) und Essigsäure (6,0 g, 0,1 mol) wurde in Toluol (50 ml) zum Rückfluss erhitzt. Anschliessend wurden innerhalb 6 Stunden 18 g Wasserphase am Wasserabscheider abgetrennt. Die organische Lösung wurde dreimal mit Wasser gewaschen (jeweils 100 ml) und am Vakuum destilliert. Die Destillation (111 bis 115°C/0,3 mbar) ergab 71,4 g Produkt, entsprechend 74% Ausbeute (bez.einges.Cyanessigsäureethylester).

Beispiel 8

(1-Cyanocyclohexyl)cyanessigsäureethylester

Eine Suspension von Kaliumcyanid (33,0 g, 0,5 mol) in Ethanol (400 ml) wurde zum Rückfluss erhitzt und anschliessend mit Cyclohexylidencyanacetat (103,3 g, 0,5 mol) versetzt. Nach einer Reaktionszeit von 45 Minuten wurde auf 60°C abgekühlt und durch Einleiten von HCl-Gas (ca. 18 g, 0,5 mol) ein pH-Wert von ca. 5 eingestellt. Das ausgefallene Kaliumchlorid wurde abfiltriert und mit Ethanol (200 ml) gewaschen. Das eingedampfte Filtrat wurde aus Ethanol umkristallisiert und ergab total 99,6 g Produkt, entsprechend einer Ausbeute von 94% (bez.einges.Cyclohexylidencyanacetat).

Beispiel 9

(1-Cyanocyclohexyl)malonsäurediethylester

Ein Gemisch von (1-Cyanocyclohexyl)cyanessigsäureethylester (2,50 g, 11,3 mmol) in Ethanol (100 ml) wurde im Autoklav bei 0°C mit HCl-Gas ca. 11 g, 0,3 mol gesättigt. Nach einer Reaktionszeit von 16 Stunden bei 0°C/2 bar wurde das Gemisch am Rotationsverdampfer eingeengt, mit Ethanol (3 ml) und Wasser (10 ml) versetzt und anschliessend bei 0°C 4 Stunden gerührt. Durch Filtration wurden 2,27 g Produkt, entsprechend einer Ausbeute von 75% (bez.einges.(1-Cyanocyclohexyl)cyanessigsäureethylester), isoliert.
Smp. 90 bis 92°C.

Beispiel 10

(1-Cyanocyclohexyl)cyanessigsäureethylester

Zu einer Suspension von Kaliumcyanid (1,33 g 20 mmol) in Ethanol (100 ml) bei 50°C wurde Ethylcyclohexylidencyanacetat (19,71 g, 100 mmol) zugegeben. Der Mischung wurde Blausäure (23,1 ml, 84 mmol) hinzugefügt, so daß der pH-Wert in einem Bereich zwischen 10,5 bis 11,5 blieb. Nach 6 Stunden bei 50°C wurde die Mischung mit Chlorwasserstoffgas (55 mmol) angesäuert. Der Niederschlag an Kaliumchlorid wurde abfiltriert und mit Ethanol (50 ml) gewaschen. Das Filtrat wurde auf 28 g eingeengt und auf 0°C abgekühlt. Die gebildeten Kristalle wurden abfiltriert, mit Ethanol gewaschen und getrocknet. Man erhielt 18,10 g des Produkts, entsprechend einer Ausbeute von 82% (bez. einges. Cyanacetat).

Beispiel 11

1-(Aminomethyl)cyclohexanessigsäurehydrochlorid

Eine Lösung von 2-Aza-4-(methoxycarbonyl)spiro[4,5]decan-3-on (1,53 g, 7,2 mmol) in 30 ml 20%iger Salzsäure wurde 24 Stunden bei Rückfluss gerührt. Die Reaktionslösung wurde abgekühlt, eingedampft, in 30 ml Wasser gelöst und nochmals eingedampft sowie getrocknet. Die erhaltene ölige Flüssigkeit wurde in 20 ml Aceton aufgeschlämmt und 5 Minuten gerührt. Danach wurde die entstandene Suspension filtriert, der Rückstand mit Aceton gewaschen und getrocknet. Man erhielt 894 mg Produkt mit einem Smp. von 114°C. Das Filtrat wurde eingedampft, der Rückstand in 10 ml 20%iger Salzsäure gelöst und während 48 Stunden rückflussiert. Die Reaktionslösung wurde abgekühlt, eingedampft, in 10 ml Wasser gelöst und wiederum eingedampft sowie getrocknet. Die erhaltene ölige Flüssigkeit wurde in 20 ml Aceton aufgeschlämmt und 5 Minuten gerührt. Danach wurde die entstandene Suspension filtriert, der Rückstand mit Aceton gewaschen und getrocknet. Man erhielt weitere 326 mg Produkt mit einem Smp. von 117°C.
Ausbeute : 81%, bez.einges.Lactam (Rohprodukt).
Smp. 114 bis 117°C.
Elementaranalyse für $C_9H_{18}NO_2Cl$ (207,7) :

ber. C 52,0% H 8,7% N 6,7%
gef. C 50,1% H 8,9% N 7,0%
Wassergehalt : 4,2%.

Beispiel 12

(1-Cyanocyclohexyl)cyanessigsäureethylester

Eine Lösung von Natriumcyanid (0,5 g, 10 mmol) in Wasser (3 ml) wurde bei 20 bis 30°C zu einer Lösung von Ethylcyclohexylidencyanacetat (2,0 g, 10 mmol) in Ethanol (5 ml) gegeben. Nach 1 Stunde bewies ein Gaschromatogramm der mit Essigsäure angesäuerten Reaktionsmischung die Bildung des Produkts (86 Flächen-% (1-Cyanocyclohexyl)cyanessigsäureethylester, 5 Flächen-% Cyclohexylidencyanacetat).

## Patentansprüche

1. 2-Aza-4-(alkoxycarbonyl)spiro[4,5]decan-3-on der allgemeinen Formel

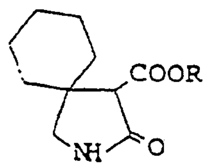

(1)

in welcher R ein niederer Alkylrest von 1 bis 4 C-Atomen bedeutet.

2. 2-Aza-4-(methoxycarbonyl)spiro[4,5]decan-3-on, 2-Aza-4-(ethoxycarbonyl)spiro[4,5]decan-3-on.

3. (1-Cyanocyclohexyl)-malonsäuredialkylester der Formel

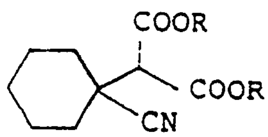

(3)

in welcher R ein niedriger Alkylrest von 1 bis 4 C-Atomen bedeutet.

4. (1-Cyanocyclohexyl)-malonsäuredimethylester, (1-Cyanocyclohexyl)-malonsäurediethylester.

5. Verfahren zur Herstellung eines 2-Aza-4-(alkoxycarbonyl)spiro[4,5]decan-3-ons gemäss mindestens einem der Patentansprüche 1 bis 2, dadurch gekennzeichnet, dass man einen Cyclohexylidenmalonsäurealkylester der allgemeinen Formel

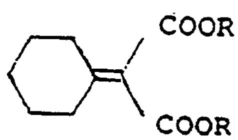

(2)

in welcher R ein niederer Alkyl von 1 bis 4 C-Atomen bedeutet, in einer ersten Stufe entweder mit Blausäure, in Gegenwart katalytischer Menge eines Alkalicyanides oder mit einer stöchiometrischen Menge Alkalicyanid, in einem Alkohol, und anschliessender Behandlung mit einer Säure in den entsprechenden (1-Cyanocyclohexyl)-malonsäuredialkylester der Formel

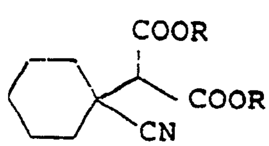

(3)

mit R wie vorstehend genannt, umsetzt und letzteren in einer zweiten Stufe durch katalytische Hydrierung in das 2-Aza-4-(alkoxycarbonyl)spiro[4,5]decan-3-on überführt.

6. Verfahren zur Herstellung eines 2-Aza-4-(alkoxycarbonyl)spiro[4,5]decan-3-ons gemäss mindestens einem der Patentansprüche 1 bis 2, dadurch gekennzeichnet, dass man ein Cyclohexylidencyanalkylat der Formel

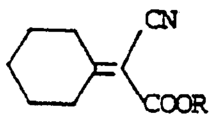

(4)

mit R wie vorstehend genannt, in einer ersten Stufe entweder mit einer stöchiometrischen Menge Alkalicyanid, in einem Alkohol, oder mit Blausäure, in Gegenwart katalytischer Menge eines Alkalicyanides in den entsprechenden (1-Cyanocyclohexyl)cyanessigsäurealkylester der Formel

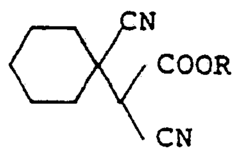

(5)

mit R wie vorstehend genannt, umsetzt, diesen in einer zweiten Stufe in einem Alkohol mit einer Säure, in den entsprechenden (1-Cyanocyclohexyl)malonsäuredialkylester der Formel

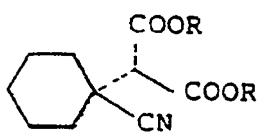

(3)

mit R wie vorstehend genannt, umsetzt, letzeren in einer dritten Stufe durch katalytische Hydrierung in das 2-Aza-4-(alkoxycarbonyl)spiro[4,5]decan-3-on überführt.

7. Verfahren nach Patentanspruch 5, dadurch gekennzeichnet, dass man die Umsetzung in der ersten Stufe entweder bei Temperaturen von 20 bis 150°C und unter sich bei Reaktionstemperatur einstellendem Druck mit HCN in einer Menge von 1 bis 20 Aequivalente, bezogen auf das Ausgangsprodukt gemäss Formel (2), und in Gegenwart von Alkalicyanid in einer Menge von 0,1 bis 50 mol%, bezogen auf das Ausgangsprodukt gemäss Formel (2), durchführt oder mit einer Menge von 1 bis 5 Aequivalenten Alkalicyanid, bezogen auf das Ausgangsprodukt gemäss Formel (2), in einem Alkohol zum Rückfluss erhitzt.

8. Verfahren nach Patentanspruch 5 bzw. 7, dadurch gekennzeichnet, dass man die katalytische Hydrierung in der zweiten Stufe bei Temperaturen von 20 bis 150°C und einem $H_2$-Druck von 1 bis 100 bar durchführt.

9. Verfahren nach Patentanspruch 6, dadurch gekennzeichnet, dass man die Umsetzung in der ersten Stufe entweder mit einer Menge von 1 bis 5 Aequivalenten Alkalicyanid, bezogen auf das Ausgangsprodukt gemäss Formel (4), in einem Alkohol zum Rückfluss erhitzt, oder bei Temperaturen von 20 bis 150°C und unter sich bei Reaktionstemperatur einstellendem Druck mit HCN in einer Menge von 1 bis 20 Aequivalente, bezogen auf das Ausgangsprodukt gemäss Formel (4), und in Gegenwart von Alkalicyanid in einer Menge von 0,1 bis 50 mol%, bezogen auf das Ausgangsprodukt gemäss Formel (4) durchführt.

10. Verfahren nach Patentanspruch 6 bzw. 9, dadurch gekennzeichnet, dass man die Umsetzung in der zweiten Stufe bei Temperaturen von –20 bis 50°C und unter einem Druck von 1 bis 10 bar mit 1 bis 100 Aequivalenten einer Säure, bezogen auf das Ausgangsprodukt gemäss Formel (5), in einem Alkohol, durchgeführt.

11. Verfahren nach mindestens einem der Patentansprüche 6, 9 und 10, dadurch gekennzeichnet, dass man die katalytische Hydrierung in der dritten Stufe bei Temperaturen von 20 bis 150°C und einem $H_2$-Druck von 1 bis 100 bar durchführt.

12. Verwendung eines 2-Aza-4-(alkoxycarbonyl)spiro[4,5]decan-3-ons zur Herstellung von 1-(Aminomethyl)cyclohexanessigsäure.

## Claims

1. 2-Aza-4-(alkoxycarbonyl)spiro[4,5]decan-3-one of the general formula

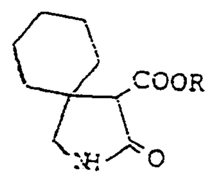

(1)

in which R is a lower alkyl residue having 1 to 4 carbon atoms.

2. 2-Aza-4-(methoxycarbonyl)spiro[4,5]decan-3-one, 2-aza-4-(ethoxycarbonyl)spiro[4,5]decan-3-one.

3. (1-Cyanocyclohexyl)malonic acid dialkyl ester of the formula

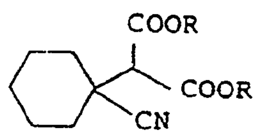

(3)

in which R is a lower alkyl residue having 1 to 4 carbon atoms.

4. (1-Cyanocyclohexyl)malonic acid dimethyl ester, (1-cyarocyclohexyl)malonic acid diethyl ester.

5. Process for the preparation of a 2-aza-4-(alkoxycarbonyl)spiro[4,5]decan-3-one according to at least one of patent claims 1 to 2, characterized in that a cyclohexylidene malonic acid alkyl ester of the general formula

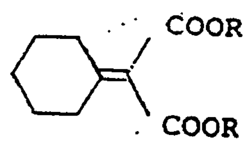

(2)

in which R is a lower alkyl having 1 to 4 carbon atoms in a first step is reacted either with hydrocyanic acid in the presence of a catalytic amount of an alkali cyanide or with a stoichiometric amount of alkali cyanide in an alcohol and is then treated with an acid to obtain the corresponding (1-cyanocyclohexyl)malonic acid dialkyl ester of the formula

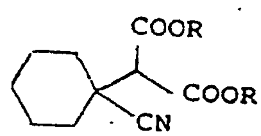

(3)

in which R is defined as above, which latter in a second step is converted into 2-aza-4-(alkoxycarbonyl)spiro[4,5]decan-3-one by catalytic hydrogenation.

6. Process for the preparation of a 2-aza-4-(alkoxycarbonyl)spiro/4,5/decan-3-one according to at least one of patent claims 1 to 2, characterized in that a cyclohexylidene cyanoalkylate of the formula

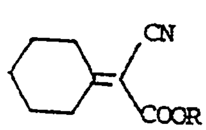

(4)

in which R is defined as above, in a first step is reacted either with a stoichiometric amount of alkali cyanide in an alcohol or with hydrocyanic acid in the presence of a catalytic amount of an alkali cyanide to obtain the corresponding (1-cyanocycyohexyy)cyanoacetic acid alkyl ester of the formula

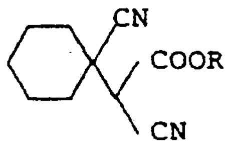

(5)

in which R is defined as above, the latter ester (5) in a second step is reacted with an acid in an alcohol to obtain the corresponding (1-cyanocyclohexyl)malonic acid dialkyl ester of the formula

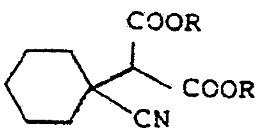

(3)

in which R is defined as above, whereupon the latter in a third step in converted into 2-aza-4-(alkoxycarbonyl)spiro[4,5]decan-3-one by catalytic hydrogenation.

7. Process according to patent claim 5, characterized in that the reaction in the first step is carried out either at temperatures of 20 to 150°C and under the pressure resulting from the reaction temperature with HCN in an amount of 1 to 20 equivalents, based on the starting product of formula (2), and in the presence of alkali cyanide in an amount of 0.1 to 50 mole%, based on the starting product of formula (2), or with an amount of 1 to 5 equivalents of alkali cyanide, based on the starting product of formula (2), in an alcohol by heating to reflux.

8. Process according to patent claims 5 and 7 resp., characterized in that the catalytic hydrogenation in the second step is carried out at temperatures of 20 to 150°C and at an $H_2$ pressure of 1 to 100 bar.

9. Process according to patent claim 6, characterized in that the reaction in the first step is carried out either with an amount of 1 to 5 equivalents of alkali cyanide, based on the starting product of formula (4), in an alcohol with heating to reflux at temperatures of 20 to 150°C and under the pressure resulting from the reaction temperature with HCN in an amount of 1 to 20 equivalents, based on the starting product of formula (4), and in the presence of alkali cyanide in an amount of 0.1 to 50 mole%, based on the starting product of formula (4).

10. Process according to patent claims 6 and 9 resp., characterized in that the reaction in the second step is carried out in an alcohol at temperatures of –20 to 50°C and at a pressure of 1 to 10 bar with 1 to 100 equivalents of an acid, based on the starting product of formula (5).

11. Process according to at least one of patent claims 6, 9 and 10, characterized in that the catalytic hydrogenation in the third step is carried out at temperatures of 20 to 150°C and at an $H_2$-pressure of 1 to 100 bar.

12. The use of a 2-aza-4-(alkoxycarbonyl)spiro[4,5]decan-3-one for the preparation of 1-(aminomethyl)cyclohexane acetic acid.

## Revendications

1. Aza-2-(alcoxycarbonyl)-4-spiro[4,5]décanone-3 de formule générale

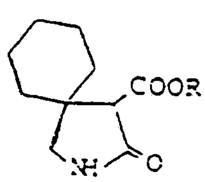

(1)

dans laquelle R représente un reste alkyle inférieur de 1 à 4 atomes de carbone.

2. Aza-2-(méthoxycarbonyl)-4-spiro[4,5]décanone-3, aza-2-(éthoxycarbonyl)-4-spiro[4,5]décanone-3.

3. (cyano-1-cyclohexyl)malonate de dialkyle de formule

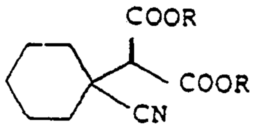

(3)

dans laquelle R représente un reste alkyle inférieur de 1 à 4 atomes de carbone.

4. (cyano-1-cyclohexyl) malonate de diméthyle, (cyano-1-cyclohexyl) malonate de diéthyle.

5. Procédé de préparation d'une aza-2-(alcoxycarbonyl)-4-spiro[4,5]décanone-3 selon au moins l'une des revendication 1 à 2, caractérisé en ce que l'on fait réagir un cyclohexylidènemalonate d'alkyle de formule générale

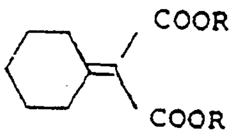

(2)

dans laquelle R représente un alkyle inférieur de 1 à 4 atomes de carbone, dans une première étape soit avec l'acide cyanhydrique, en présence d'une quantité catalytique d'un cyanure alcalin, soit avec une quantité stoechiométrique de cyanure alcalin, dans un alcool, puis l'on traite avec un acide pour obtenir le (cyano-1-cyclohexyl)malonate de dialkyle correspondant de formule

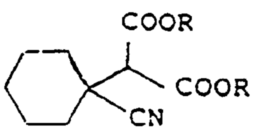

(3)

dans laquelle R a la signification donnée précédemment, et on convertit celui-ci dans une deuxième étape, par hydrogénation catalytique, en la aza-2-(alcoxycarbonyl)-4-spiro[4,5]décanone-3.

6. Procédé de préparation d'une aza-2-(alcoxycarbonyl)-4-spiro[4,5]décanone-3 selon au moins l'une des revendications 1 à 2, caractérisé en ce que l'on fait réagir un cyclohexylidènecyanacétate d'alkyle de formule

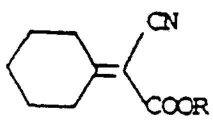

(4)

dans laquelle R est tel que défini ci-dessus, dans une première étape soit avec une quantité stoechiométrique de cyanure alcalin, dans un alcool, soit avec l'acide cyanhydrique, en présence d'une quantité catalytique d'un cyanure alcalin pour former le (cyano-1-cyclohexyl)cyanacétate d'alkyle de formule

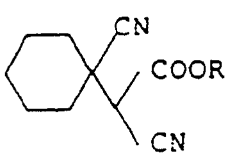

(5)

dans laquelle R est tel que défini ci-dessus, on convertit celui-ci dans une seconde étape dans un alcool avec un acide, en le (cyano-1-cyclohexyl)malonate de dialkyle de formule

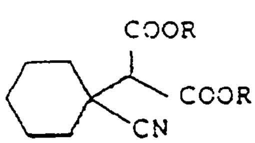

(3)

dans laquelle R est tel que défini ci-dessus, on convertit ce lui-ci dans une troisième étape par hydrogénation catalytique en la aza-2-(alcoxycarbonyl)-4-spiro[4,5]décanone-3.

7. Procédé selon la revendication 5, caractérisé en ce que soit l'on réalise la conversion de la première étape à des températures de 20 à 150°C et sous la pression qui s'établit à la température de la réaction avec HCN en une quantité de 1 à 20 équivalents, par rapport au produit de départ selon la formule (2), et en présence d'un cyanure alcalin en une quantité de 0,1 à 50 mol%, par rapport au produit de départ selon la formule (2), soit l'on chauffe à reflux noirs un alcool avec une quantité de 1 à 5 équivalents de cyanure alcalin par rapport au produit de départ selon la formule (2).

8. Procédé selon la revendication 5 ou 7, caractérisé en ce que l'on réalise l'hydrogénation catalytique de la deuxième étape à des températures de 20 à 150°C et sous une pression de $H_2$ de 1 à 100 bars.

9. Procédé selon la revendication 6, caractérisé en ce que l'on réalise la conversion de la première étape soit avec une quantité de 1 à 5 équivalents de cyanure alcalin, par rapport au produit de départ selon la formule (4), en chauffant à reflux dans un alcool, soit à des températures de 20 à 150°C et sous la pression qui s'établit à la température de la réaction avec HCN en une quantité de 1 à 20 équivalents par rapport au produit de départ selon la formmule (4), et en présence de cyanure alcalin en une quantité de 0,1 à 50 mol% par rapport au produit de départ selon la formule (4).

10. Procédé selon la revendication 6 ou 9, caractérisé en ce que l'on réalise la conversion de la deuxième étape à des températures de −20 à 50°C et sous une pression de 1 à 10 bars avec 1 à 100 équivalents d'un acide, par rapport au produit de départ selon la formule (5), dans un alcool.

11. Procédé selon au moins l'une des revendications 6, 9 et 10, caractérisé en ce que l'on réalise l'hydrogénation catalytique de la troisième étape à des températures de 20 à 1500°C et sous une pression de $H_2$ de 1 à 100 bars.

12. Utilisation d'une aza-2-(alcoxycarbonyl)-4-spiro [4,5] décanone-3 pour la préparation de l'acide (aminométhyl)-1-cyclohexane-acétique.